(19) Europäisches Patentamt
European Patent Office
Office européen des brevets

(11) Numéro de publication : **0 684 233 A1**

# (12) DEMANDE DE BREVET EUROPEEN

(21) Numéro de dépôt : **95400777.9**

(22) Date de dépôt : **07.04.95**

(51) Int. Cl.[6] : **C07D 211/28,** C07D 211/32, C07D 211/20, C07D 211/36, C07D 413/04, // (C07D413/04, 271:00, 211:00)

(30) Priorité : **12.04.94 FR 9404289**

(43) Date de publication de la demande : **29.11.95 Bulletin 95/48**

(84) Etats contractants désignés : **AT BE CH DE DK ES FR GB GR IE IT LI LU NL PT SE**

(71) Demandeur : **SYNTHELABO**
**22, Avenue Galilée**
**F-92350 Le Plessis Robinson (FR)**

(72) Inventeur : **Mallart, Sergio**
**17ter avenue du Maréchal Joffre**
**F-91400 Orsay (FR)**
Inventeur : **Lassalle, Gilbert**
**53 avenue Victor Hugo**
**F-92140 Clamart (FR)**
Inventeur : **Galtier, Daniel**
**16 rue Francis Poulenc**
**F-78280 Guyancourt (FR)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Dérivés de l'acide pipécolique, leur préparation et leur utilisation comme intermédiaires de synthèse.**

(57) Composés de formule (1)

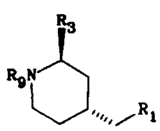

(1)

dans laquelle $R_1$ représente soit un atome d'hydrogène, soit un groupe méthyle, $R_3$ représente soit un groupe formyle, soit un groupe hydroxyméthyle, soit un groupe $-COCH_2R_4$ où $R_4$ est choisi parmi les groupes hydroxy, $(C_1-C_4)$alcoxycarbonyle et (1,1-diméthyléthyl)diméthylsilyloxy, soit un groupe $-CONHSO_2R_5$ où $R_5$ est choisi parmi les groupes $(C_1-C_4)$alkyle et phényl$(C_1-C_4)$alkyle, soit un groupe $-CH_2N(OH)COR_6$ où $R_6$ est choisi parmi les groupes amino, $(C_1-C_4)$alkyle et phényl$(C_1-C_4)$alcoxy, soit un groupe $-XCO_2C_2H_5$ où X est choisi parmi les groupes 1,2-dioxoéthane-1,2-diyle et dicarbonyle, soit un groupe $-CHR_7(CH_2)_nOH$ où $R_7$ est un groupe hydroxy et n = 1 à 4, soit un groupe $-CR_8{=}NOH$ où $R_8$ est un atome d'hydrogène ou un groupe $(C_1-C_4)$alkyle, soit un groupe 1*H*-imidazol- 4(5)-yle, soit un groupe 4,5-dihydro-1*H*-imidazol-4(5)-yle, soit un groupe 1*H*-pyrazol-4-yle, soit un groupe 1*H*-pyrazol-5-yle, soit un groupe isoxazol-4-yle, soit un groupe isoxazol-5-yle, soit un groupe oxazol-4-yle, soit un groupe oxazol-5-yle, soit un groupe 4,5-dihydrooxazol-4-yle, soit un groupe 4,5-dihydrooxazol-5-yle, soit un groupe 1*H*-1,2,3-triazol-4 (5)-yle, soit un groupe 1,2,4-oxadiazol-3-yle, soit un groupe 1,2,4-oxadiazol-5-yle, soit un groupe 1,3,4-oxadiazol-2-yle, éventuellement substitués par un groupe hydroxy et/ou par un groupe ZH où Z est choisi parmi les atomes d'oxygène, de soufre et le groupe imino et
$R_9$ représente un atome d'hydrogène ou un groupe (1,1-diméthyléthoxy)carbonyle ainsi que leurs sels d'addition à des acides forts. Intermédiaires de synthèse.

EP 0 684 233 A1

Jouve, 18, rue Saint-Denis, 75001 PARIS

La présente invention a pour objet des dérivés de l'acide pipécolique, leur préparation et leur utilisation comme intermédiaires de synthèse.

Les composés de l'invention répondent à la formule (1)

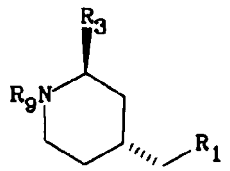

(1)

dans laquelle

$R_1$ représente soit un atome d'hydrogène, soit un groupe méthyle,

$R_3$ représente soit un groupe formyle, soit un groupe hydroxyméthyle, soit un groupe $-COCH_2R_4$ où $R_4$ est choisi parmi les groupes hydroxy, $(C_1-C_4)$alcoxycarbonyle et (1,1-diméthyléthyl)diméthylsilyloxy, soit un groupe $-CONHSO_2R_5$ où $R_5$ est choisi parmi les groupes $(C_1-C_4)$alkyle et phényl$(C_1-C_4)$alkyle, soit un groupe $-CH_2N(OH)COR_6$ où $R_6$ est choisi parmi les groupes amino, $(C_1-C_4)$alkyle et phényl$(C_1-C_4)$alcoxy, soit un groupe $-XCO_2C_2H_5$ où X est choisi parmi les groupes 1,2-dioxoéthane-1,2-diyle et dicarbonyle, soit un groupe $-CHR_7(CH_2)_nOH$ où $R_7$ est un groupe hydroxy et n = 1 à 4, soit un groupe $-CR_8=NOH$ où $R_8$ est un atome d'hydrogène ou un groupe $(C_1-C_4)$alkyle, soit un groupe 1*H*-imidazol-4(5)-yle, soit un groupe 4,5-dihydro-1*H*-imidazol- 4(5)-yle, soit un groupe 1*H*-pyrazol-4-yle, soit un groupe 1*H*-pyrazol-5-yle, soit un groupe isoxazol-4-yle, soit un groupe isoxazol-5-yle, soit un groupe oxazol-4-yle, soit un groupe oxazol-5-yle, soit un groupe 4,5-dihydrooxazol-4-yle, soit un groupe 4,5-dihydrooxazol-5-yle, soit un groupe 1*H*-1,2,3-triazol-4 (5)-yle, soit un groupe 1,2,4-oxadiazol-3-yle, soit un groupe 1,2,4-oxadiazol-5-yle, soit un groupe 1,3,4-oxadiazol-2-yle, éventuellement substitués par un groupe hydroxy et/ou par un groupe ZH où Z est choisi parmi les atomes d'oxygène, de soufre et le groupe imino et

$R_9$ représente soit un atome d'hydrogène soit un groupe (1,1-diméthyléthoxy)carbonyle.

La configuration préférentielle du groupe pipéridinyle est [2*R*, 4*R*].

Les composés peuvent exister sous forme de bases libres ou de sels d'addition à des acides forts.

Conformément à l'invention on peut synthétiser les composés de formule (1) selon des méthodes qui sont décrites dans la littérature ou qui sont connues de l'homme du métier.

Les composés de départ sont disponibles dans le commerce ou décrits dans la littérature ou peuvent être préparés selon des méthodes qui y sont décrites ou qui sont connues de l'homme du métier.

Ainsi le (2*R*-*trans*)-1-[(1,1-diméthyléthoxy)carbonyl]-4-méthyl-β-oxopipéridine-2-propanoate d'éthyle est préparé selon une méthode analogue à celle décrite par D. W. Brooks, Angew. Chem., Int., Ed. Engl., (1979), 18, 72.

Le (2*R*-*trans*)-2-(3-amino-1,2,4-oxadiazol-5-yl)-4-méthylpipéridine-1-carboxylate de 1,1-diméthyléthyle est préparé selon une méthode analogue à celle décrite par L. J. Street et coll. J. Med. Chem., (1990), 33, 2690.

La préparation de l'ester 1-(1,1-diméthyléthylique) de l'acide (2*R*-*trans*)-4-méthylpipéridine-1,2-dicarboxylique est décrite dans la demande de brevet européen no 565396.

Les exemples suivants illustrent de manière non exhaustive la préparation de certains composés selon l'invention.

Les microanalyses élémentaires et les spectres IR et RMN confirment la structure des composés obtenus.

Les numéros entre parenthèses dans les titres des exemples renvoient au tableau donné plus loin. Ce tableau illustre les structures chimiques et les propriétés physiques de quelques composés selon l'invention.

Exemple 1 (composé n° 1)

chlorhydrate de (2*R*-*trans*)-4-méthyl-β-oxopipéridine-2-propanoate d'éthyle

1.1. ester 1-(1,1-diméthyléthylique) de l'acide (2*R*-*trans*)-4-méthylpipéridine-1,2-dicarboxylique

On place 0,51 g (3 mmoles) de (2*R*-*trans*)-4-méthylpipéridine-2-carboxylate d'éthyle dans 10 ml de dichlorométhane et on ajoute, sous azote, à 0 °C, 654 mg (3 mmoles) de dicarbonate de bis(1,1-diméthyléthyle). On laisse remonter à la température ambiante pendant une nuit. On évapore et on reprend le résidu dans 4 ml d'éthanol et 3 ml d'une solution d'hydroxyde de sodium 1 N. On laisse une nuit à la température ambiante puis on ajoute de nouveau 3 ml d'une solution d'hydroxyde de sodium 1 N. Ensuite, on évapore, on reprend le résidu par de l'eau et on lave avec de l'éther. On acidifie le milieu avec de l'acide chlorhydrique 1 N puis on extrait deux fois par de l'éther. On rince les phases organiques par 10 ml d'une solution saturée en chlorure de sodium et on sèche sur du sulfate de magnésium.

On obtient 0,68 g de produit.

Point de fusion = 87-90 °C Rendement = 97 %

1.2. (2*R*-*trans*)-1-[(1,1-diméthyléthoxy)carbonyl]-4-méthyl-β-oxopipéridine-2-propanoate d'éthyle

A une solution de 486 mg (2 mmoles) d'ester 1-(1,1-diméthyléthylique) de l'acide (2*R*-*trans*)-4-méthylpipéridine-1,2-dicarboxylique dans 4 ml de tétrahydrofurane, on ajoute 360 mg (2,2 mmoles) de *N,N'*-carbonyldiimidazole et on agite le milieu réactionnel pendant 6 heures à 20 °C. Ensuite on ajoute 570 mg (2,2 mmoles) de sel de magnésium du malonate d'éthyle et on laisse sous agitation pendant 16 heures à 20 °C. On dilue alors le milieu réactionnel avec 25 ml d'éther et on le lave successivement par 10 ml d'une solution 1 N d'acide chlorhydrique puis par 10 ml d'une solution saturée de chlorure de sodium. On recueille la phase organique, on la sèche sur sulfate de magnésium, on la filtre, on la concentre et on purifie le résidu par chromatographie sur colonne de gel de silice en éluant par un mélange acétate d'éthyle:hexane (10:90).

On obtient 520 mg de produit sous forme d'une huile incolore.

$[\alpha]_D^{20}$ = + 81,6 ° (c = 1; chloroforme)

1.3. chlorhydrate de (2*R*-*trans*)-4-méthyl-β-oxopipéridine-2-propanoate d'éthyle

On dilue 520 mg (1,6 mmoles) de (2*R*-*trans*)-1-[(1,1-diméthyléthoxy)carbonyl]-4-méthyl-β-oxopipéridine-2-propanoate d'éthyle dans 2 ml d'éther, on refroidit le mélange à 0 °C et on ajoute 2 ml d'une solution 4 N d'acide chlorhydrique dans le dioxane. On laisse le milieu réactionnel sous agitation pendant 1 heure à 0 °C, on laisse la température du mélange remonter à la température ambiante, on poursuit l'agitation pendant 4 heures à 20 °C puis on concentre le mélange à sec.

On obtient 400 mg de produit sous forme d'un solide blanc.

RMN (200 MHz - $CDCl_3$) : 10-9 (2H, large) ; 4,75 (1H, large) ; 4,25 (2H, q, 7,2 Hz) ; 3,73 (1H, large) ; 3,45 (2H, large) ; 2,25 (1H, large) ; 2-1,5 (5H) ; 1,3 (3H, t, 7,2 Hz) ; 1,01 (3H, d, 7 Hz)

Exemple 2 (composé n° 2)

(2*R*-*trans*)-2-(hydroxyméthyl)-4-méthylpipéridine-1-carboxylate de 1,1-diméthyléthyle

On refroidit à 0 °C une solution de 23 g (100 mmoles) de l'ester 1-(1,1-diméthyléthylique) de l'acide (2*R*-*trans*)-4-méthylpipéridine-1,2-dicarboxylique dans 100 ml de tétrahydrofurane, on ajoute 30 ml (300 mmoles) d'une solution de complexe de diméthylsulfure de borane dans 50 ml de tétrahydrofurane, on laisse la température du mélange remonter à 20 °C et on le laisse sous agitation pendant 16 heures à 20 °C. Ensuite on refroidit le milieu réactionnel à 0 °C, on ajoute doucement 50 ml de méthanol, on évapore à sec, on reprend le résidu huileux dans 20 ml de méthanol et on l'évapore de nouveau à sec. On recommence l'opération trois fois et finalement on reprend le résidu dans 50 ml de toluène et on évapore à sec.

On obtient 21,5 g de produit que l'on utilise tel quel dans l'étape suivante.

$[\alpha]_D^{20}$ = + 37,2 ° (c = 1,3; chloroforme)

Exemple 3 (composé no 3)

(2*R*-*trans*)-2-formyl-4-méthylpipéridine-1-carboxylate de 1,1-diméthyléthyle

On refroidit à -78 °C une solution de 13,9 g (110 mmoles) de chlorure d'oxalyle dans 100 ml de dichlorométhane et on ajoute lentement 19,5 g (250 mmoles) de diméthylsulfoxyde en solution dans 100 ml de dichlorométhane. On agite le mélange à -78 °C pendant 15 minutes, on ajoute 21,5 g (100 mmoles) de (2*R*-*trans*)-2-(hydroxyméthyl)-4-méthylpipéridine-1-carboxylate de 1,1-diméthyléthyle en solution dans 50 ml de dichlorométhane et on laisse sous agitation pendant 1 heure à -78 °C. Ensuite on ajoute 50 g (500 mmoles) de triéthylamine en solution dans 100 ml de dichlorométhane, on laisse le mélange sous agitation pendant 30 minutes à -78 °C puis on le réchauffe lentement à 20 °C. Ensuite on dilue le milieu réactionnel avec 300 ml d'éther et 300 ml d'une solution 1 N d'acide chlorhydrique, on sépare les phases, on recueille la phase organique et on la lave successivement par 200 ml d'une solution 1 N d'hydrogénocarbonate de sodium et par 200 ml d'une solution saturée de chlorure de sodium. On la sèche sur sulfate de sodium, on la filtre et on l'évapore à sec. On purifie l'huile colorée ainsi obtenue par chromatographie sur colonne de gel de silice en éluant par un mélange acétate d'éthyle:cyclohexane (10:90).

On obtient 17 g de produit.

$[\alpha]_D^{20}$ = + 26,7 ° (c = 1; chloroforme)

Exemple 4 (composé n° 4)

(2*R-trans*)-2-[(hydroxyimino)méthyl]-4-méthylpipéridine

4.1. (2*R-trans*)-2-[(hydroxyimino)méthyl]-4-méthylpipéridine-1-carboxylate de 1,1-diméthyléthyle

On solubilise 750 mg (10,7 mmoles) de chlorhydrate d'hydroxylamine dans 2 ml d'eau et on ajoute 1,95 g (8,6 mmoles) de (2*R-trans*)-2-formyl-4-méthylpipéridine-1-carboxylate de 1,1-diméthyléthyle en solution dans 2 ml de tétrahydrofurane puis 570 mg (5,4 mmoles) de carbonate de sodium en solution dans 1,5 ml d'eau. On laisse le milieu réactionnel sous agitation pendant 2,5 heures à 20 °C, on l'acidifie à pH 4 avec une solution 1 N de bisulfate de sodium et on l'extrait par deux fois 50 ml d'éther. On recueille la phase organique, on la sèche sur sulfate de sodium, on la concentre et on la filtre. On obtient une huile que l'on purifie par chromatographie sur colonne de gel de silice en éluant par un mélange acétate d'éthyle:hexane (10:90).

On obtient 1,23 g du produit sous forme trans et 0,61 g de produit sous forme cis.

4.2. chlorhydrate de (2*R-trans*)-2-[(hydroxyimino)méthyl]-4-méthylpipéridine

On obtient le composé à partir du (2*R-trans*)-2-[(hydroxyimino)méthyl]-4-méthylpipéridine-1-carboxylate de 1,1-diméthyléthyle selon la méthode décrite dans l'exemple 1.3.

On obtient le produit sous forme d'un solide jaune pâle. Point de fusion = 175 °C

Exemple 5 (composé n° 5)

(2*R-trans*)-2-[[(aminocarbonyl)hydroxyamino]méthyl]-4-méthylpipéridine

5.1. (2*R-trans*)-2-[(hydroxyamino)méthyl]-4-méthylpipéridine-1-carboxylate de 1,1-diméthyléthyle

A 1,2 g (5 mmoles) d'un mélange cis/trans de (2*R-trans*)-2-[(hydroxyimino)méthyl]-4-méthylpipéridine-1-carboxylate de 1,1-diméthyléthyle, en solution dans 10 ml d'acide acétique, à 20 °C, on ajoute par petites portions 310 mg (5 mmoles) de cyanoborohydrure de sodium et on laisse le milieu réactionnel pendant 3 heures sous agitation à 20 °C. Ensuite on le dilue par 50 ml d'éther et 15 ml d'eau puis on ajuste le pH à 8-9 par addition de bicarbonate de sodium par petites portions. On sépare les phases, on extrait la phase aqueuse par deux fois 50 ml de dichlorométhane et on rassemble les phases organiques. On les sèche sur sulfate de sodium, on les filtre et on les concentre à sec.

On obtient 1,18 g de produit sous forme d'une huile que l'on utilise telle quelle dans l'étape suivante.

5.2. (2*R-trans*)-2-[[(aminocarbonyl)hydroxyamino]méthyl]-4-méthylpipéridine-1-carboxylate de 1,1-diméthyléthyle

A une solution de 1,03 g (4,22 mmoles) de (2*R-trans*)-2-[(hydroxyamino)méthyl]-4-méthylpipéridine-1-carboxylate de 1,1-diméthyléthyle dans 5 ml de dioxane, on ajoute une solution de 550 mg (8,5 mmoles) de cyanate de sodium dans 5 ml d'eau puis 8,9 ml d'une solution 1 N d'acide chlorhydrique. On agite le milieu réactionnel pendant 16 heures à 20 °C puis on le dilue par 20 ml d'eau et 50 ml de dichlorométhane. On sépare les phases, on extrait la phase aqueuse par deux fois 25 ml de dichlorométhane et on rassemble les phases organiques. On les lave par une solution aqueuse à 5 % de bicarbonate de soude, on les sèche sur sulfate de sodium, on les filtre et on les évapore à sec. On purifie le résidu obtenu par chromatographie sur colonne de gel de silice en éluant par un mélange méthanol:dichlorométhane (5:95).

On obtient 800 mg de produit que l'on utilise tel quel dans l'étape suivante.

5.3. chlorhydrate du (2*R-trans*)-2-[[(aminocarbonyl)hydroxyamino]méthyl]-4-méthylpipéridine (1:1)

On prépare le composé selon la méthode décrite dans l'exemple 1.3 à partir du (2*R-trans*)-2-[[aminocarbonyl)hydroxyamino] méthyl]-4-méthylpipéridine-1-carboxylate de 1,1-diméthyléthyle.

On obtient le produit sous forme d'un solide incolore.

Point de fusion = 150 °C

Exemple 6 (composé n° 6)

(2*R-trans*)-2-[(acétylhydroxyamino)méthyl]-4-méthylpipéridine

6.1. (2*R-trans*)-2-[(acétylhydroxyamino)méthyl]-4-méthylpipéridine-1-carboxylate de 1,1-diméthyléthyle

On traite, à 0 °C, une solution de 500 mg (2 mmoles) de (2*R-trans*)-2-(hydroxyamino)méthyl]-4-méthylpipéridine-1-carboxylate de 1,1-diméthyléthyle dans 5 ml de dichlorométhane, par 160 μl (2 mmoles) de pyridine et 160 μl (2,2 mmoles) de chlorure d'acétyle. On laisse le milieu réactionnel sous agitation pendant 30 minutes à 0 °C, on le dilue par 50 ml de dichlorométhane, puis on le lave successivement par une solution aqueuse 0,3 M de bisulfate de sodium puis par une solution saturée de chlorure de sodium. On recueille la phase aqueuse, on la lave sur sulfate de magnésium, on la filtre et on l'évapore à sec.

On obtient 478 mg de produit sous forme d'un solide incolore.

6.2. chlorhydrate de (2*R*-*trans*)-2-[(acétylhydroxyamino) méthyl]-4-méthylpipéridine

On le prépare selon la méthode de l'exemple 1.3 à partir du (2*R*-*trans*)-2-[(acétylhydroxyamino)méthyl]-4-méthylpipéridine-1-carboxylate de 1,1-diméthyléthyle.

RMN (200 MHz - $CDCl_3$) : 10 (2H, large) ; 8,6 (1H, large) ; 7,9 (1H, large) ; 4,3 (1H, m) ; 3,75 (1H, m) ; 3,25 (2H, large) ; 2,2 (1H, m) ; 2,1 (3H, s) ; 2-1,4 (5H) ; 1,01 (3H, d, 7 Hz)

Exemple 7 (composés n° 7 et 8)

(2*R*-*trans*)-2-[2-[[(1,1-diméthyléthyl)diméthylsilyl]oxy]-1-oxoéthyl]-4-méthylpipéridine

7.1. (2*R*-*trans*)-2-éthényl-4-méthylpipéridine-1-carboxylate de 1,1-diméthyléthyle

On refroidit à -78 °C une suspension de 3,57 g (10 mmoles) de bromure de méthyltriphénylphosphonium dans 10 ml de tétrahydrofurane puis on ajoute 10 ml d'une solution 1 M de 1,1,1, 3,3,3-hexaméthyldisilazide de potassium dans le tétrahydrofurane. On agite le milieu réactionnel pendant 1 heure à cette température, on ajoute une solution de 2,27 g (10 mmoles) de (2*R*-*trans*)-2-formyl-4-méthylpipéridine-1-carboxylate de 1,1-diméthyléthyle dans 5 ml de tétrahydrofurane puis on laisse le mélange sous agitation à une température comprise entre -78 °C à +20 °C pendant 3 heures. Ensuite on hydrolyse le milieu réactionnel avec 10 ml d'une solution 1 N d'acide chlorhydrique et on l'extrait par 3 fois 25 ml d'éther. On rassemble les phases organiques, on les lave successivement par 10 ml d'une solution saturée d'hydrogénocarbonate de sodium et par 10 ml d'une solution saturée de chlorure de sodium, on les sèche sur sulfate de sodium, on les filtre et on les évapore à sec. On purifie le résidu obtenu par chromatographie sur colonne de gel de silice en éluant par un mélange acétate d'éthyle:cyclohexane (5:95).

On obtient 1,73 g de produit que l'on utilise tel quel dans les étapes suivantes.

$[\alpha]_D^{20}$ = + 22,4 ° (c = 1,7; chloroforme)

7.2. (2*R*-*trans*)-2-(1,2-dihydroxyéthyl)-4-méthylpipéridine-1-carboxylate de 1,1-diméthyléthyle

A 900 mg (4 mmoles) de (2*R*-*trans*)-2-éthényl-4-méthylpipéridine-1-carboxylate de 1,1-diméthyléthyle en solution dans 2 ml d'acétone et 2 ml d'eau, à 20 °C, on ajoute 515 mg (4,4 mmoles) de *N*-méthylmorpholine *N*-oxyde, 10 mg de tétraoxyde d'osmium et 50 µl de pyridine. On laisse le mélange sous agitation à cette température pendant 5 heures puis on dilue le milieu réactionnel avec 10 ml d'eau et on l'extrait par trois fois 25 ml d'éther. On recueille la phase organique, on la sèche sur sulfate de sodium, on la filtre et on la concentre. On purifie le résidu ainsi obtenu par chromatographie sur colonne de gel de silice en éluant par un mélange acétate d'éthyle:cyclohexane (40:60).

On obtient 500 mg du diastéréoisomère le moins polaire,

$[\alpha]_D^{20}$ = + 53,7 ° (c = 0,98; dichlorométhane)

Point de fusion = 81 °C

et 320 mg du diastéréoisomère le plus polaire.

$[\alpha]_D^{20}$ = + 38,6 ° (c = 0,99; dichlorométhane)

Point de fusion = 75 °C

7.3. (2*R*-*trans*)-2-[2-[[(1,1-diméthyléthyl)diméthylsilyl]oxy]-1-oxoéthyl]-4-méthylpipéridine-1-carboxylate de 1,1-diméthyléthyle (composé n° 7)

A 414 mg (2 mmoles) de (2*R*-*trans*)-2-(1,2-dihydroxyéthyl)-4-méthylpipéridine-1-carboxylate de 1,1-diméthyléthyle sous forme de mélange de diastéréoisomères et 300 mg (2,1 mmoles) de chlorure de (1,1-diméthyléthyle)diméthylsilane en solution dans 4 ml de dichlorométhane, on ajoute 323 µl (2,1 mmoles) de triéthylamine et 10 mg de 4-diméthylaminopyridine. On laisse le mélange sous agitation pendant 4 heures à 20 °C, on le dilue avec 25 ml d'éther puis on le lave successivement avec 10 ml d'une solution 0,1 N d'acide chlorhydrique et 10 ml d'une solution saturée d'hydrogénocarbonate de sodium. On recueille la phase organique, on la sèche sur sulfate de sodium, on la filtre et on l'évapore à sec. On obtient une huile incolore que l'on dissout dans 2 ml de dichlorométhane. On refroidit le mélange à -78 °C et on ajoute une solution, préalablement refroidie à -78 °C, de 266,7 mg (2,1 mmoles) de chlorure d'oxalyle et de 390 mg (5 mmoles) de diméthylsulfoxyde dans le dichlorométhane. On laisse le milieu réactionnel sous agitation à cette température, on ajoute 1,54 ml de triéthylamine et on laisse la température du mélange revenir à 20 °C. On dilue alors le milieu réactionnel par 50 ml d'éther et on ajoute 10 ml d'une solution 0,5 N d'acide chlorhydrique. On sépare les phases, on recueille la phase organique, on la lave successivement par 10 ml d'une solution 1 M d'hydrogénocarbonate de sodium et 10 ml d'une solution saturée de chlorure de sodium, on la sèche sur sulfate de sodium, on la filtre et on la concentre à sec. On purifie le résidu ainsi obtenu par chromatographie sur colonne de gel de silice en éluant par un mélange acétate d'éthyle:cyclohexane (5:95).

On obtient 400 mg de produit.

$[\alpha]_D^{20}$ = + 13,2 ° (c = 0,98; chloroforme)

7.4. chlorhydrate de (2*R*-*trans*)2-[2-[[(1,1-diméthyléthyl) diméthylsilyl]oxy]-1-oxoéthyl]-4-méthylpipéridine (composé n° 8)

On prépare le composé à partir du (2*R*-*trans*)-2-[2-[[(1,1-diméthyléthyl)diméthylsilyl]oxy]-1-oxoéthyl]-4-méthylpipéridine-1-carboxylate de 1,1-diméthyléthyle selon la méthode décrite dans l'exemple 1.3.

On obtient le produit sous forme d'une huile incolore.

Exemple 8 (composés n° 9 et 10)

chlorhydrate de (2*R*-*trans*)-2-[[(éthylsulfonyl)amino] carbonyl]-4-méthylpipéridine

8.1. (2*R*-*trans*)-2-[[éthylsulfonyl)amino]carbonyl]-4-méthylpipéridine-1-carboxylate de 1,1-diméthyléthyle

Dans 10 ml de dichlorométhane, on fait réagir 486 mg (2 mmoles) de l'ester 1-(1,1-diméthyléthylique) de l'acide (2*R*-*trans*)-4-méthylpipéridine-1,2-dicarboxylique, 229 mg (2,1 mmoles) d'éthanesulfonamide, 460 mg (2,3 mmoles) de chlorhydrate de 1,3-dicyclohexylcarbodiimide et 538 mg (4,4 mmoles) de 4-diméthylaminopyridine. On laisse le milieu réactionnel pendant 36 heures à la température ambiante puis on le dilue par de l'acétate d'éthyle. On le lave successivement par une solution aqueuse d'acide chlorhydrique 0,2 N, par de l'eau et par une solution saturée de chlorure de sodium. On le sèche sur sulfate de magnésium et on l'évapore à sec. On reprend le résidu obtenu dans du dichlorométhane et on le purifie par chromatographie sur colonne de gel de silice en éluant par du dichlorométhane.

On obtient 0,5 g de produit.

Point de fusion = 131-133 °C

8.2. chlorhydrate de (2*R*-*trans*)-2-[[éthylsulfonyl)amino] carbonyl]-4-méthylpipéridine

On prépare le composé selon la méthode décrite dans l'exemple 1.3 à partir du (2*R*-*trans*)-2-[[(éthylsulfonyl)amino] carbonyl]-4-méthylpipéridine-1-carboxylate de 1,1-diméthyléthyle.

Point de fusion = 103 °C

Exemple 9 (composés n° 11 et 12)

chlorhydrate de (2*R*-*trans*)-4-méthyl-2-[[[(phénylméthyl) sulfonyl]amino]carbonyl]pipéridine

9.1. (2*R*-*trans*)-4-méthyl-2-[[[(phénylméthyl)sulfonyl] amino]carbonyl]pipéridine-1-carboxylate de 1,1-diméthyléthyle

Dans 10 ml de dichlorométhane, on fait réagir 486 mg (2 mmoles) de l'ester 1-(1,1-diméthyléthylique) de l'acide (2*R*-*trans*)-4-méthylpipéridine-1,2-dicarboxylique, 360 mg (2,1 mmoles) de benzèneméthanesulfonamide, 460 mg (2,3 mmoles) de chlorhydrate de *N*-éthyl-*N'*-(3-diméthylaminopropyl)carbodiimide et 538 mg (4,4 mmoles) de 4-diméthylaminopyridine, On laisse le milieu réactionnel pendant 36 heures à la température ambiante puis on le dilue par de l'acétate d'éthyle. On le lave successivement par une solution aqueuse d'acide chlorhydrique 0,2 N, par de l'eau et par une solution saturée de chlorure de sodium. On le sèche sur sulfate de magnésium et on l'évapore à sec. On reprend le résidu obtenu dans 100 ml d'éther diéthylique et on cristallise le produit dans du pentane.

On obtient 0,58 g de produit sous forme de cristaux blancs.

Point de fusion = 137-139 °C

9.2. chlorhydrate de (2*R*-*trans*)-4-méthyl-2-[[[(phénylméthyl) sulfonyl]amino]carbonyl]pipéridine

On le prépare à partir du (2*R*-*trans*)-4-méthyl-2-[[[(phénylméthyl)sulfonyl]amino]carbonyl]pipéridine-1-carboxylate de 1,1-diméthyléthyle selon la méthode décrite dans l'exemple 1.3.

Point de fusion = 130 °C

Exemple 10 (composés n° 13 et 14)

chlorhydrate de (2*R*-*trans*)-2-(3-amino-1,2,4-oxadiazol-5-yl)-4-méthylpipéridine

10.1. (2*R*-*trans*)-2-(3-amino-1,2,4-oxadiazol-5-yl)-4-méthylpipéridine-1-carboxylate de 1,1-diméthyléthyle

On solubilise 323 mg (10 mmoles) de sodium dans 10 ml d'éthanol, on ajoute 850 mg de tamis moléculaire (4 Å) puis 1,05 g (4 mmoles) d'hémisulfate d'hydroxyguanidine. On laisse le milieu réactionnel sous agitation pendant 30 minutes à la température ambiante, on ajoute 542 mg (2 mmoles) de (2*R*-*trans*)-1-[(1,1-diméthyléthoxy)carbonyl]-4-méthyl-pipéridine-2-carboxylate d'éthyle et on porte le mélange à la température de reflux pendant 3 heures. Ensuite on le filtre et on le concentre à sec. On reprend le résidu par 50 ml d'un mélange dichlorométhane:eau (1:1), on récupère la phase organique, on la sèche sur sulfate

de magnésium et on la concentre à sec. On purifie le résidu par chromatographie sur colonne de gel de silice en éluant par un mélange méthanol:dichlorométhane (5:95).

RMN (200 MHz - $CDCl_3$) : 5,6-5,4 (1H, large) ; 5 (2H, large) ; 4,1 (1H, large) ; 3,0 (1H, large) ; 2,3 (1H, m) ; 1,6-1,3 (4H, m) ; 1,5 (9H, s) ; 0,95 (3H, d, 7 Hz)

10.2. (2*R-trans*)-2-(3-amino-1,2,4-oxadiazol-5-yl)-4-méthylpipéridine

On le prépare à partir du (2*R-trans*)-2-(3-amino-1,2,4-oxadiazol-5-yl)-4-méthylpipéridine-1-carboxylate de 1,1-diméthyléthyle selon la méthode décrite dans l'exemple 1.3

On obtient 308 mg de produit sous la forme d'une huile incolore.

RMN (200 MHz - $CDCl_3$) : 11 (2H, large) ; 8-7 (3H, large) ; 4,9 (1H, t, 4Hz) ; 3,4 (2H, m) ; 2,3 (1H, m) ; 2-1,4 (m, 4H) ; 1,01 (3H, d, 7 Hz)

Exemple 11 (composés n° 15 et 16)

chlorhydrate de (2*R-trans*)-2-(5-mercapto-1,3,4-oxadiazol-2-yl)-4-méthyl-pipéridine

11.1. (2*R-trans*)-1-[(1,1-diméthyléthoxy)carbonyl]-4-méthylpipéridine-2-carboxyhydrazide

On porte à la température de reflux pendant 18 heures, une solution contenant 2,71 g (10 mmoles) de (2*R-trans*)-1-[(1,1-diméthyléthoxy)carbonyl]-4-méthyl-pipéridine-2-carboxylate d'éthyle et 1 g (20 mmoles) d'hydrate d'hydrazine dans 10 ml de méthanol. On concentre le milieu à sec, et on purifie le résidu par chromatographie sur colonne de gel de silice en éluant par un mélange dichlorométhane:méthanol (96:4).

On obtient 2,23 g de produit sous forme d'une huile incolore.

11.2. (2*R-trans*)-2-(5-mercapto-1,3,4-oxadiazol-2-yl)-4-méthyl-piperidine-1-carboxylate de 1,1-diméthyléthyle (composé n° 15)

A une solution de 1,12 g (4,33 mmoles) de (2*R-trans*)-1-[(1,1-diméthyléthoxy)carbonyl]-4-méthyl-pipéridine-2-carboxyhydrazide, on ajoute à 0 °C, 0,77 g (10 mmoles) de disulfure de carbone et 0,26 g (4,6 mmoles) de potasse en pastilles. On porte le mélange à la température de reflux pendant 7 heures puis on le concentre à sec. On reprend le résidu par un mélange 1:1 d'éther et d'une solution 1 N d'acide chlorhydrique, on recueille la phase organique, on la sèche sur sulfate de magnésium, on la filtre et on la concentre à sec sous pression réduite. On purifie le résidu obtenu par chromatographie sur colonne de gel de silice en éluant par un mélange dichlorométhane:méthanol (95:5).

On obtient 0,93 g de produit sous forme d'une huile incolore.

11.3. chlorhydrate de (2*R-trans*)-2-(5-mercapto-1,3,4-oxadiazol-2-yl)-4-méthyl-pipéridine (composé n° 16)

On prépare le composé selon la méthode décrite dans l'exemple 1.3.

On obtient le produit sous forme d'un solide blanc.

Point de fusion = 165-170 °C

Exemple 12 (composé n° 17)

chlorhydrate de (2*R-trans*)-2-(5-mercapto-1,3,4-oxadiazol-2-yl)-4-éthyl-pipéridine

On prépare le composé selon la méthode décrite dans l'exemple 11 à partir du (2*R-trans*)-1-[(1,1-diméthyléthoxy)carbonyl]-4-éthyl-pipéridine-2-carboxyhydrazide.

| | | | | | |
|---|---|---|---|---|---|
| Analyse : | calc. | C=43,28 ; | H=6,46 ; | N=16,82 ; | Cl=14,19 |
| | mes. | C=43,56 ; | H=6,49 ; | N=16,28 ; | Cl=14,57 |

Tableau

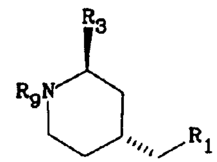

(1)

| No | $R_1$ | $R_3$ | $R_9$ | Sel | Caractéristiques |
|---|---|---|---|---|---|
| 1 | -H | $-COCH_2CO_2C_2H_5$ | -H | HCl | RMN : 10-9 (2H, large) ; 4,75 (1H, large) ; 4,25 (2H, q, 7,2 Hz) ; 3,73 (1H, large) ; 3,45 (2H, large) ; 2,25 (1H, large) ; 2-1,5 (5H) ; 1,3 (3H, t, 7,2 Hz) ; 1,01 (3H, d, 7 Hz) |
| 2 | -H | $-CH_2OH$ | $-CO_2C(CH_3)_3$ | - | $[\alpha]_D^{20}$ = + 37,2 ° (c = 1,3; chloroforme) |
| 3 | -H | -CHO | $-CO_2C(CH_3)_3$ | - | $[\alpha]_D^{20}$ = + 26,7 ° (c = 1; chloroforme) |
| 4 | -H | -CH=NOH | -H | HCl | Point de fusion = 175 °C |
| 5 | -H | $-CH_2N(OH)CONH_2$ | -H | HCl | Point de fusion = 150 °C |
| 6 | -H | $-CH_2N(OH)COCH_3$ | -H | HCl | RMN : 10 (2H, large) ; 8,6 (1H, large) ; 7,9 (1H, large) ; 4,3 (1H, m) ; 3,75 (1H, m) ; 3,25 (2H, large) ; 2,2 (1H, m) ; 2,1 (3H, s) ; 2-1,4 (5H) ; 1,01 (3H, d, 7 Hz) |
| 7 | -H | $-COCH_2OSi(CH_3)_2C(CH_3)_3$ | $-CO_2C(CH_3)_3$ | - | $[\alpha]_D^{20}$ = + 13,2 ° (c = 0,98; chloroforme) |
| 8 | -H | $-COCH_2OSi(CH_3)_2C(CH_3)_3$ | -H | HCl | huile incolore |
| 9 | -H | $-CONHSO_2C_2H_5$ | $-CO_2C(CH_3)_3$ | - | Point de fusion = 131-133 °C |

| No | $R_1$ | $R_3$ | $R_9$ | Sel | Caractéristiques |
|---|---|---|---|---|---|
| 10 | -H | $-CONHSO_2C_2H_5$ | -H | HCl | Point de fusion = 103 °C |
| 11 | -H | $-CONHSO_2CH_2C_6H_5$ | $-CO_2C(CH_3)_3$ | - | Point de fusion = 137-139 °C |
| 12 | -H | $-CONHSO_2CH_2C_6H_5$ | -H | HCl | Point de fusion = 130 °C |
| 13 | -H | 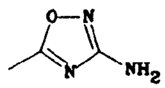 | $-CO_2C(CH_3)_3$ | - | RMN : 5,6-5,4 (1H, large) ; 5 (2H, large) ; 4,1 (1H, large) ; 3,0 (1H, large) ; 2,3 (1H, m) ; 1,6-1,3 (4H, m) ; 1,5 (9H, s) ; 0,95 (3H, d, 7 Hz) |
| 14 | -H | 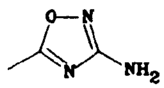 | -H | HCl | RMN : 11 (2H, large) ; 8-7 (3H, large) ; 4,9 (1H, t, 4Hz) ; 3,4 (2H, m) ; 2,3 (1H, m) ; 2-1,4 (m, 4H) ; 1,01 (3H, d, 7 Hz) |
| 15 | -H | 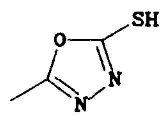 | $-CO_2C(CH_3)_3$ | - | Huile incolore |
| 16 | -H | 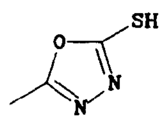 | -H | HCl | Point de fusion = 165-170 °C |
| 17 | $-CH_3$ | 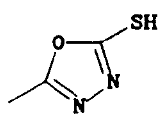 | -H | HCl | calc. C=43,28 ; H=6,46 ; N=16,82 ; Cl=14,19<br>mes. C=43,56 ; H=6,49 ; N=16,28 ; Cl=14,57 |

Les spectres RMN sont mesurés à 200 MHz dans $CDCl_3$

Les composés selon l'invention sont utiles par exemple pour la synthèse de composés de formule (I)

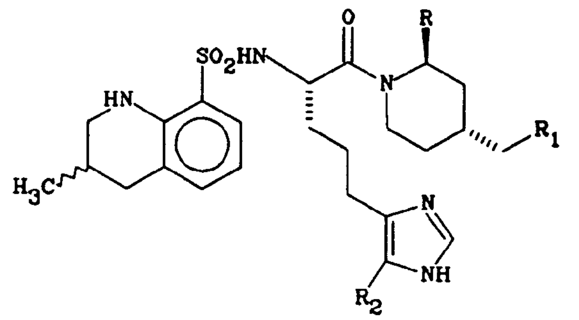

(I)

dans laquelle

R représente soit un groupe $-COCH_2R_4$ où $R_4$ est choisi parmi les groupes hydroxy et ($C_1$-$C_4$)alcoxycarbonyle, soit un groupe $-CONHSO_2R_5$ où $R_5$ est choisi parmi les groupes ($C_1$-$C_4$)alkyle et phényl($C_1$-$C_4$)alkyle, soit un groupe $-CH_2N(OH)COR_6$ où $R_6$ est choisi parmi les groupes amino, ($C_1$-$C_4$)alkyle et phényl($C_1$-$C_4$)alcoxy, soit un groupe $-XCO_2C_2H_5$ où X est choisi parmi les groupes 1,2-dioxoéthane-1,2-diyle et dicarbonyle, soit un groupe $-CHR_7(CH_2)_nOH$ où $R_7$ est un groupe hydroxy et n = 1 à 4, soit un groupe $-CR_5{=}NOH$ où $R_5$ est un atome d'hydrogène ou un groupe ($C_1$-$C_4$)alkyle, soit un groupe 1*H*-imidazol-4(5)-yle, soit un groupe 4,5-dihydro-1*H*-imidazol-4(5)-yle, soit un groupe 1*H*-pyrazol-4-yle, soit un groupe 1*H*-pyrazol-5-yle, soit un groupe isoxazol-4-yle, soit un groupe isoxazol-5-yle, soit un groupe oxazol-4-yle, soit un groupe oxazol-5-yle, soit un groupe 4,5-dihydrooxazol-4-yle, soit un groupe 4,5-dihydrooxazol-5-yle, soit un groupe 1*H*-1,2,3-triazol-4(5)-yle, soit un groupe 1,2,4-oxadiazol-3-yle, soit un groupe 1,2,4-oxadiazol-5-yle, soit un groupe 1,3,4-oxadiazol-2-yle, éventuellement substitués par un groupe hydroxy et/ou par un groupe ZH où Z est choisi parmi les atomes d'oxygène, de soufre et le groupe imino,

$R_1$ et $R_2$ représentent chacun indépendamment l'un de l'autre, soit un atome d'hydrogène, soit un groupe méthyle.

On peut par exemple, préparer les composés de formule (I) par réaction de l'acide (*S*)-α-[[(3-méthyl-1,2,3,4-tétrahydroquinoléin-8-yl)sulfonyl]amino]-1-(triphénylméthyl)-1*H*-imidazole-4-pentanoïque avec le composé de formule (1) correspondant.

Les exemples 13 à 15 qui suivent illustrent cette synthèse sans la limiter.

## Exemple 13

chlorhydrate de [2*R*-[1(*S*), 2α, 4β]]-1-[5-(1*H*-imidazol-4-yl)-2-[[(3-méthyl-1,2,3,4-tétrahydroquinoléin-8-yl)sulfonyl]amino]-1-oxopentyl]-4-méthyl-β-oxopipéridine-2-propanoate d'éthyle

13.1. acide (*S*)-α-[[(3-méthyl-1,2,3,4-tétrahydroquinoléin-8-yl)sulfonyl]amino]-1-(triphénylméthyl)-1*H*-imidazole-4-pentanoïque

13.1.1. acide 3-méthyl-1,2,3,4-tétrahydroquinoléine-8-sulfonique

On place 20 g (90 mmoles) d'acide 3-méthylquinoléine-8-sulfonique dans un mélange contenant 200 ml d'eau et 25 ml d'acide chlorhydrique 12 N. On ajoute 6 g de catalyseur au rhodium à 5 % et on chauffe à 70 °C pendant 16 heures. On rince le catalyseur à l'eau chaude et on évapore. On reprend le résidu par 50 ml d'éthanol et on le sèche sur du pentoxyde de phosphore, à 50 °C.

On obtient 14 g de produit.

Point de fusion = 255 °C (décomposition)

13.1.2. (*S*)-α-[[(3-méthyl-1,2,3,4-tétrahydroquinoléin-8-yl)sulfonyl]amino]-1-(triphénylméthyl)-1*H*-imidazole-4-pentanoate d'éthyle

On dissout 10,46 g (40 mmoles) de triphénylphosphine dans 60 ml de dichlorométhane, à 0 °C. Sous azote, à 0 °C, on ajoute goutte à goutte 3,56 ml (44 mmoles) de chlorure de sulfuryle. Ensuite, on laisse remonter à la température ambiante et on ajoute, en 10 minutes, 4,56 g (20 mmoles) d'acide 3-méthyl-1,2,3,4-tétrahydroquinoléine-8-sulfonique dissous dans un mélange contenant 100 ml de dichlorométhane et 2,78 ml de triéthylamine. On laisse sous agitation pendant environ une heure à la température ambiante puis on verse le milieu réactionnel sur 500 ml de pentane. On filtre, on évapore le filtrat et on reprend le résidu par environ 500 ml de pentane. On obtient 5 g de produit sous forme d'une huile que l'on reprend dans 50 ml de dichlorométhane. Sous azote, à 0 °C, on ajoute 35 ml de cette dernière solution à une solution de 4 g (7,72 mmoles) de chlorhydrate de (*S*)-α-amino-1-(triphénylméthyl)-1*H*-imidazole-4-pentanoate d'éthyle dans 50 ml de dichlorométhane. On laisse sous agitation pendant une heure puis on lave successivement par 50 ml d'acide chlorhydrique 1 N, 50 ml d'eau, 50 ml d'une solution saturée en hydrogénocarbonate de sodium, 50 ml d'eau et finalement par 50 ml d'une solution saturée en chlorure de sodium. On sèche sur du sulfate de magnésium et on purifie le résidu par chromatogra-phie sur colonne de gel de silice en éluant par un mélange dichlorométhane/méthanol (97/3).

On obtient 4,7 g de produit.

Point de fusion = 70-80 °C

13.1.3. acide (*S*)-α-[[(3-méthyl-1,2,3,4-tétrahydroquinoléin-8-yl)sulfonyl]amino]-1-(triphénylméthyl)-1*H*-imidazole-4-pentanoïque

On place 4,5 g (6,5 mmoles) de (*S*)-α-[[(3-méthyl-1,2,3,4-tétrahydroquinoléin-8-yl)sulfonyl]amino]-1-(triphénylméthyl)-1H-imidazole-4-pentanoate d'éthyle dans 80 ml de benzène, on refroidit à 0 °C et on laisse barboter pendant une heure de l'acide chlorhydrique gazeux. On laisse ensuite encore 30 minutes à 0 °C et on évapore. On reprend le résidu dans 10 ml de chloroforme et on ajuste le pH à 8-9 avec de l'ammoniac en solution dans du dichlorométhane. On purifie par chromatographie sur colonne de gel de silice en éluant par un gradient dichlorométhane/méthanol (95/5 à 90/10).

On obtient 3,5 g de produit que l'on utilise tel quel dans l'étape suivante.

Point de fusion = 125-145 °C

13.2. [2*R*-[1(*S*), 2α, 4β]]-1-[2-[[(3-méthyl-1,2,3,4-tétrahydroquinoléin-8-yl)sulfonyl]amino]-1-oxo-5-[1-(triphénylméthyl)-1*H*-imidazol-4-yl]pentyl]-4-méthyl-β-oxopipéridine-2-propanoate d'éthyle

A 400 mg (1,5 mmoles) de chlorhydrate de (2*R*-*trans*)-4-méthyl-β-oxopipéridine-2-propanoate d'éthyle, on ajoute 845 mg (1,3 mmoles) de l'acide (*S*)-α-[[(3-méthyl-1,2,3,4-tétrahydroquinoléin-8-yl)sulfonyl]amino]-1-(triphénylméthyl)-1*H*-imidazole-4-pentanoïque et 5 ml de dichlorométhane. On refroidit la solution à 0 °C, on ajoute 730 μl (4,2 mmoles) de *N*,*N*-diisopropyléthylamine, puis 572 mg (1,3 mmoles) d'hexafluorophosphate de [(benzotriazol-1-yl)oxy]tris(diméthylamino) phosphonium, on laisse le mélange sous agitation pendant 1 heure à 0 °C, on laisse la température remonter à la température ambiante et on poursuit l'agitation pendant 5 heures à 20 °C. Ensuite on dilue le milieu réactionnel par 50 ml d'acétate d'éthyle et on le lave successivement par 20 ml d'une solution 0,5 N d'acide chlorhydrique, 10 ml d'eau, 20 ml d'une solution saturée d'hydrogénocarbonate de sodium et 10 ml d'une solution saturée de chlorure de sodium. On recueille la phase organique, on la sèche sur sulfate de sodium et on la filtre. On purifie le résidu par chromatographie sur colonne de gel de silice en éluant par un mélange éthanol:dichlorométhane (2:98).

On obtient 780 mg de produit que l'on utilise tel quel dans l'étape suivante.

13.3. chlorhydrate de [2*R*-[1(*S*), 2α, 4β]]-1-[5-(1*H*-imidazol-4-yl)-2-[[(3-méthyl-1,2,3,4-tétrahydroquinoléin-8-yl) sulfonyl]amino]-1-oxopentyl]-4-méthyl-β-oxopipéridine-2-propanoate d'éthyle

On dissout 780 mg (0,93 mmole) de [2*R*-[1(*S*), 2α, 4β]]-1-[2-[[(3-méthyl-1,2,3,4-tétrahydroquinoléin-8-yl)sulfonyl]amino]-1-oxo-5-[1-(triphénylméthyl)-1*H*-imidazol-4-yl]pentyl]-4-méthyl-β-oxopipéridine-2-propanoate d'éthyle dans 5 ml de dichlorométhane, on refroidit la solution à 0 °C et on ajoute 200 μl d'anisole et 2,5 ml d'acide trifluoracétique. On agite le mélange à une température comprise entre 0 et 20 °C pendant 5 heures, on l'hydrolyse à 0 °C avec 20 ml d'une solution saturée d'hydrogénocarbonate de sodium et on le neutralise par du bicarbonate de sodium solide. On sépare les phases, on extrait la phase aqueuse avec 2 fois 25 ml de dichlorométhane et on rassemble les phases organiques. Ensuite on les sèche sur sulfate de sodium, on les filtre et on les concentre. On obtient un résidu huileux que l'on purifie par chromatographie sur colonne de gel de silice en éluant par un mélange éthanol:dichlorométhane (5:95).

On obtient 390 mg de base sous forme d'une mousse incolore. On prépare le chlorhydrate en solubilisant 390 mg (0,59 mmole) de produit par sonication dans un équivalent d'une solution 0,1 N d'acide chlorhydrique puis en lyophilisant la solution.

On obtient 415 mg de chlorhydrate sous forme d'un solide incolore.

$[\alpha]_D^{20}$ = + 76 ° (c = 0,5; méthanol)

Point de fusion = 91 °C

Exemple 14

chlorhydrate de [2*R*-[1(*S*), 2α, 4β]]-2-(3-hydroxyisoxazol-5-yl)-1-[5-(1*H*-imidazol-4-yl)-2-[[(3-méthyl-1,2,3,4-tétrahydroquinoléin-8-yl)sulfonyl]amino]-1-oxopentyl]-4-méthylpipéridine

A une solution de 280 mg (0,5 mmole) de [2*R*-[1(*S*), 2α, 4β]]-1-[5-(1*H*-imidazol-4-yl)-2-[[(3-méthyl-1,2,3,4-tétrahydroquinoléin-8-yl)sulfonyl]amino]-1-oxopentyl]-4-méthyl-β-oxopipéridine-2-propanoate d'éthyle dans 1 ml de méthanol, on ajoute simultanément 0,5 ml d'une solution de soude 1 N et 35 mg (0,5 mmole) de chlorhydrate d'hydroxylamine en solution dans 0,5 ml d'une solution de soude 1 N. Ensuite on agite le milieu réactionnel pendant 1 heure à 20 °C, on le jette dans un mélange contenant 5 ml d'une solution d'acide chlorhydrique concentrée et 5 g de glace et on agite pendant 16 heures à 20 °C. On concentre à sec, on reprend le résidu dans 5 ml d'eau et on le purifie par chromatographie sur colonne de gel de silice en éluant par un mélange d'acide chlorhydrique 0,2 N et d'acétonitrile (gradient d'acétonitrile 0 à 100 % en 100 minutes). Finalement on recueille les fractions contenant le produit, on les évapore à sec et on reprend le résidu ainsi obtenu par 5 ml d'eau et on le lyophilise.

On obtient 100 mg de produit.

$[\alpha]_D^{20}$ = + 94 ° (c = 0,36; chloroforme)

Point de fusion = 130-135 °C

Exemple 15

chlorhydrate de [2*R*-[1(*S*), 2α, 4β]]-2-(3-amino-1,2,4-oxadiazol-5-yl)-1-[5-(1*H*-imidazol-4-yl)-2-[[(3-méthyl-1,2,3,4-tétrahydroquinoléin-8-yl)sulfonyl]amino]-1-oxopentyl]-4-méthylpipéridine

On fait réagir l'acide (*S*)-α-[[(3-méthyl-1,2,3,4-tétrahydroquinoléin-8-yl)sulfonyl]amino]-1-(triphénylméthyl)-1*H*-imidazole-4-pentanoïque avec le (2*R*-*trans*)-2-(3-amino-1,2,4-oxadiazol-5-yl)-4-méthylpipéridine-1-carboxylate de 1,1-diméthyléthyle, selon la méthode décrite dans l'exmple 10 puis on déprotège le composé obtenu et on prépare le chlorhydrate selon la méthode décrite dans l'exemple 10.

On obtient 150 mg de produit sous forme d'un solide incolore.

$[\alpha]_D^{20}$ = 165 ° (c = 0,2 ; méthanol)

Point de fusion = 165 °C

Les composés de formule (I) présentent une activité antithrombotique et sont décrits dans les demandes françaises 94.04290 et 94.04291.

## Revendications

1. Composés de formule (1)

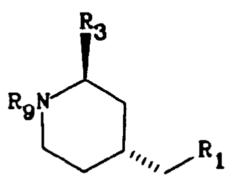

(1)

dans laquelle

$R_1$ représente soit un atome d'hydrogène, soit un groupe méthyle,

$R_3$ représente soit un groupe formyle, soit un groupe hydroxyméthyle, soit un groupe $-COCH_2R_4$ où $R_4$ est choisi parmi les groupes hydroxy, ($C_1$-$C_4$)alcoxycarbonyle et (1,1-diméthyléthyl)diméthylsilyloxy, soit un groupe $-CONHSO_2R_5$ où $R_5$ est choisi parmi les groupes ($C_1$-$C_4$)alkyle et phényl($C_1$-$C_4$) alkyle, soit un groupe $-CH_2N(OH)COR_5$ où $R_5$ est choisi parmi les groupes amino, ($C_1$-$C_4$)alkyle et phényl($C_1$-$C_4$)alcoxy, soit un groupe $-XCO_2C_2H_5$ où X est choisi parmi les groupes 1,2-dioxoéthane-1,2-diyle et dicarbonyle, soit un groupe $-CHR_7(CH_2)_nOH$ où $R_7$ est un groupe hydroxy et n = 1 à 4, soit un groupe $-CR_8$=NOH où $R_8$ est un atome d'hydrogène ou un groupe ($C_1$-$C_4$)alkyle, soit un groupe 1*H*-imidazol- 4(5)-yle, soit un groupe 4,5-dihydro-1*H*-imidazol- 4(5)-yle, soit un groupe 1*H*-pyrazol-4-yle, soit un groupe 1*H*-pyrazol-5-yle, soit un groupe isoxazol-4-yle, soit un groupe isoxazol-5-yle, soit un groupe oxazol-4-yle, soit un groupe oxazol-5-yle, soit un groupe 4,5-dihydrooxazol-4-yle, soit un groupe 4,5-dihydrooxazol-5-yle, soit un groupe 1*H*-1,2,3-triazol-4 (5)-yle, soit un groupe 1,2,4-oxadiazol-3-yle, soit un groupe 1,2,4-oxadiazol-5-yle, soit un groupe 1,3,4-oxadiazol-2-yle, éventuellement substitués par un groupe hydroxy et/ou par un groupe ZH où Z est choisi parmi les atomes d'oxygène, de soufre et le groupe imino et

$R_9$ représente un atome d'hydrogène ou un groupe (1,1-diméthyléthoxy)carbonyle ainsi que leurs sels d'addition à des acides forts.

2. Composés selon la revendication 1, caractérisés en ce que la configuration préférentielle du groupe pipéridinyle est [2*R*, 4*R*].

3. Utilisation des composés selon l'une quelconque des revendications 1 et 2 en particulier pour la synthèse de composés de formule (I)

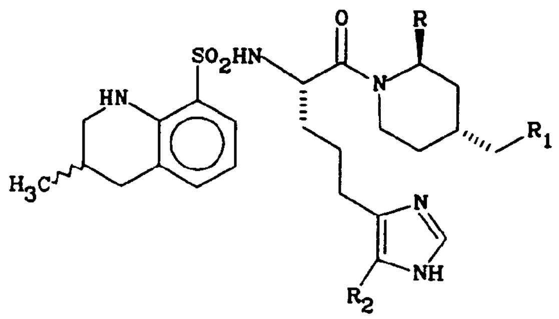

(I)

dans laquelle
R représente soit un groupe $-COCH_2R_4$ où $R_4$ est choisi parmi les groupes hydroxy et $(C_1-C_4)$alcoxycarbonyle, soit un groupe $-CONHSO_2R_5$ où $R_5$ est choisi parmi les groupes $(C_1-C_4)$alkyle et phényl$(C_1-C_4)$alkyle, soit un groupe $-CH_2N(OH)COR_6$ où $R_6$ est choisi parmi les groupes amino, $(C_1-C_4)$alkyle et phényl$(C_1-C_4)$alcoxy, soit un groupe $-XCO_2C_2H_5$ où X est choisi parmi les groupes 1,2-dioxoéthane-1,2-diyle et dicarbonyle, soit un groupe $-CHR_7(CH_2)_nOH$ où $R_7$ est un groupe hydroxy et n = 1 à 4, soit un groupe $-CR_8=NOH$ où $R_6$ est un atome d'hydrogène ou un groupe $(C_1-C_4)$alkyle, soit un groupe 1*H*-imidazol-4(5)-yle, soit un groupe 4,5-dihydro-1*H*-imidazol-4(5)-yle, soit un groupe 1*H*-pyrazol-4-yle, soit un groupe 1*H*-pyrazol-5-yle, soit un groupe isoxazol-4-yle, soit un groupe isoxazol-5-yle, soit un groupe oxazol-4-yle, soit un groupe oxazol-5-yle, soit un groupe 4,5-dihydrooxazol-4-yle, soit un groupe 4,5-dihydrooxazol-5-yle, soit un groupe 1*H*-1,2,3-triazol-4(5)-yle, soit un groupe 1,2,4-oxadiazol-3-yle, soit un groupe 1,2,4-oxadiazol-5-yle, soit un groupe 1,3,4-oxadiazol-2-yle, éventuellement substitués par un groupe hydroxy et/ou par un groupe ZH où Z est choisi parmi les atomes d'oxygène, de soufre et le groupe imino et
$R_1$ et $R_2$ représentent chacun indépendamment l'un de l'autre, soit un atome d'hydrogène, soit un groupe méthyle.

Office européen des brevets

# RAPPORT DE RECHERCHE EUROPEENNE

Numero de la demande
EP 95 40 0777

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | JOURNAL OF HETEROCYCLIC CHEMISTRY, vol.9, no.4, 1972, PROVO US pages 875 - 878 R. CAHILL ET AL. * exemple 2d * | 1 | C07D211/28<br>C07D211/32<br>C07D211/20<br>C07D211/36<br>C07D413/04<br>//(C07D413/04, 271:00,211:00) |
| X,D | EP-A-0 565 396 (SYNTHELABO) * Interm. (IX) pour Composés No. 28, 34 * | 1 | |
| A | EP-A-0 008 746 (MITSUBISHI CHEMICAL INDUSTRIES LTD.) * page 12, ligne 11 * | 1 | |
| A,P | EP-A-0 614 986 (SYNTHELABO) * abrégé * | 1-3 | |

DOMAINES TECHNIQUES RECHERCHES (Int.Cl.6)
C07D

Le présent rapport a été établi pour toutes les revendications

| Lieu de la recherche | Date d'achèvement de la recherche | Examinateur |
|---|---|---|
| BERLIN | 4 Octobre 1995 | Frelon, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)